(19) Europäisches Patentamt
European Patent Office
Office européen des brevets

(11) **EP 3 446 684 B1**

(12) **EUROPEAN PATENT SPECIFICATION**

(45) Date of publication and mention
of the grant of the patent:
**17.11.2021 Bulletin 2021/46**

(21) Application number: **17813385.6**

(22) Date of filing: **15.06.2017**

(51) Int Cl.:
*A61K 31/135* (2006.01)      *A61K 31/197* (2006.01)
*A61K 31/4184* (2006.01)      *A61K 9/00* (2006.01)
*A61K 9/20* (2006.01)

(86) International application number:
**PCT/JP2017/022119**

(87) International publication number:
**WO 2017/217491 (21.12.2017 Gazette 2017/51)**

(54) **ORALLY DISINTEGRATING TABLET**

SCHMELZTABLETTE

COMPRIMÉS ORODISPERSIBLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.06.2016   JP 2016120056**

(43) Date of publication of application:
**27.02.2019 Bulletin 2019/09**

(73) Proprietor: **TOWA PHARMACEUTICAL CO., LTD.
Kadoma-shi,
Osaka 571-8580 (JP)**

(72) Inventors:
• **SAEKI, Isamu**
**Kadoma-shi,
Osaka 5718580 (JP)**
• **MATSUSHIMA, Yuki**
**Kadoma-shi,
Osaka 5718580 (JP)**
• **KABASHIMA, Hiroko**
**Kadoma-shi,
Osaka 5718580 (JP)**
• **OKUDA, Yutaka**
**Kadoma-shi,
Osaka 5718580 (JP)**

(74) Representative: **Isarpatent
Patent- und Rechtsanwälte Barth
Charles Hassa Peckmann & Partner mbB
Friedrichstrasse 31
80801 München (DE)**

(56) References cited:
**EP-A1- 1 203 580      EP-A1- 2 196 221
EP-A1- 2 810 659      WO-A1-00/54752
JP-A- 2015 218 322**

• **Anonymous: "Pharmaceutical dosage forms:
tablets", 2008, Informa Healthcare, New York,
USA, XP002789571, vol. 1 * pages 77-78 ***
• **MITSUO MATSUMOTO et al.: Yakuzaigaku
Mannual, 1989, page 49, XP009510851,**
• **Sujitha H. Shivani: "REVIEW ARTICLE ON
MICROPARTICLES", Int. . J. of Pharmacy and
Analytical Research, 1 January 2015
(2015-01-01), pages 302-309, XP55669628,
Retrieved from the Internet:
URL:http://www.ijpar.com/sites/default/fil
es/articles/IJPAR-15%20SHIVANI_302-309.pdf
[retrieved on 2020-02-18]**

**Description**

TECHNICAL FIELD

The present invention relates to the field of tablet production, in particular, the field of orally disintegrating tablet, and to an orally disintegrating tablet having a sufficient hardness yet rapidly disintegrating in the presence of water, and a method of production thereof.

BACKGROUND ART

An orally disintegrating tablet is a tablet that is designed so that the entire tablet disintegrates with saliva within a short of time when put in the mouth, and has been developed as a form of tablet that can be easily taken by elders and infants (Patent documents 1-3).

An orally disintegrating tablet must have a property that it rapidly disintegrates when exposed to water (saliva) in the oral cavity. On the other hand, it must be able to keep the integrity of its initial form as a tablet without fracturing, crushing or wearing due to external forces like vibrations, physical shocks, or pressures in various foreseeable conditions of its handling in the course where it is produced in the form of tablet, packaged, shipped, transported in diverse circumstances, stored in medical facilities or pharmacies, pushed out of the package (blister pack) there and handed to a patient in a separate package, or pushed out of the blister pack by the patient before being taken. For this reason, it must have sufficient hardness so that it withstands such external forces. However, it is not easy to adequately realize both of those properties, i.e., easy disintegration and sufficient hardness. This is because: while the more firmly are combined the components of the tablet such as a pharmaceutically active ingredient, excipients and other additives, the higher becomes its hardness, which very likely tends to make the tablet the less disintegrable; and conversely, while the weaker is the combination of those components, the higher becomes its disintegrability, which then leads to insufficient hardness and thus renders the tablet prone to fracturing and wearing. Therefore, developments have been conducted aimed to provide an orally disintegrating tablet which possesses the both rather incompatible properties in good balance, or to improve the performance of an orally disintegrating tablet by enhancing its disintegrability while achieving sufficient hardness (Patent documents 4-8).

On the other hand, the fast-paced aging of society is a factor that increases the need for the preparation form of orally disintegrating tablet. Thus, there is a probability that the form of orally disintegrating tablet could come to be demanded all the more frequently for those oral preparations widely used for various existing drugs. In order to be able to meet such a social demand when it arises, it is desirable that the form of disintegrating tablet can be surely utilized not only for some particular drugs but also for other wide variety of ones, and further that an orally disintegrating tablet can be provided exhibiting enhanced disintegration speed.

PRIOR ART DOCUMENTS

PATENT DOCUMENTS

Patent document 1: JPS58-24410
Patent document 2: JPH06-502194
Patent document 3: WO95/20380
Patent document 4: JP4551627
Patent document 5: JP5584509
Patent document 6: JP5062761
Patent document 7: JP5062872
Patent document 8: JP4446177

EP-A-2810659 describes an orally disintegrating tablet as a compression molding product of a mixture comprising at least a pregelatinized starch and granules containing a povidone, titanium oxide and caffeine.

EP-A-1203580 describes quickly disintegrating solid preparations which contain: a) an active ingredient; b) D-mannitol having an average particle size of 30 $\mu$m to 300 $\mu$m; c) a disintegrating agent; and d) celluloses.

"Pharmaceutical dosage forms: tablets", 2008, Informa Healthcare, New York, USA, vol. 1, pages 77-78 teaches that the fluid bed granulation parameters such as the binder addition rate/method, and drying time, and fluidization behavior for the powder bed are critical to flowability.

# SUMMARY OF INVENTION

## TECHNICAL PROBLEM

Against the above background, the present inventors fixed attention on the necessity of a means that enables, with drugs to be orally administered, quick formulation of orally disintegrating tablet having advantageous properties that can bring about speedier disintegration in the presence of water while having a sufficient hardness.

Thus, it is an objective of the present invention to find out a universal means usable to formulate various orally administered drugs into the form of such an orally disintegrating tablet that is advantageous both in its hardness and disintegrability, and to enable speedy provision of orally disintegrating tablets having such advantageous properties.

## SOLUTION TO PROBLEM

As a result of studies toward the above objective, the present inventors discovered that granules containing an active ingredient (pharmaceutically active ingredient-containing granules), if the granules have a certain specific particle-size distribution and physical property, produce a tablet thereupon exhibiting advantageous disintegrability even having a sufficient hardness, and through further studies, have completed the present invention. Thus, the present invention provides what follows.

1. An orally disintegrating tablet as a compression molding product of a mixture comprising at least a disintegrant, and granules A containing a pharmaceutically active ingredient, wherein the proportion of granules A in the orally disintegrating tablet is at least 25 weight %, and
wherein

(a) the mean particle size of granules A is not greater than 300 $\mu$m, and said mean particle size of granules A is the 50% particle size ($D_{50}$) in particle size distribution (weight-based) determined by the sieving method,
(b) the particle distribution index for granules A is 3.0 or less, and particle distribution index = ($D_{50}/D_{10}$ + $D_{90}/D_{50}$)/2, and particle size distribution (weight-based) is determined by the sieving method,
(c) the angle of repose of granules A is not greater than 38°, and said angle of repose is a value determined according to "Determination of the repose angle", the Japanese Pharmacopoeia, 17th edition, and
(d) the aspect ratio of granules A is at least 0.7, and said aspect ratio is an index representing the shape of a particle and determined by ISO 9276-6 as "Feret's distance/ISO maximum length" of a particle, and

wherein the content of the pharmaceutically active ingredient in the granules A represents at least 70 weight % of the weight of the plain granules of granules A.

2. The orally disintegrating tablet according to 1 above, wherein the mean particle size of granules A is not greater than 250 $\mu$m.

3. The orally disintegrating tablet according to 1 or 2 above, wherein the particle distribution index for granules A is 2.5 or less.

4. A method for production of an orally disintegrating tablet comprising compression molding a mixture comprising at least a disintegrant, and granules A containing a pharmaceutically active ingredient, wherein the proportion of granules A in the orally disintegrating tablet is at least 25 weight %, and
wherein

(a) the mean particle size of granules A is not greater than 300 $\mu$m, and said mean particle size of granules A is the 50% particle size ($D_{50}$) in particle size distribution (weight-based) determined by the sieving method,
(b) the particle distribution index for granules A is 3.0 or less, and particle distribution index = ($D_{50}/D_{10}$ + $D_{90}/D_{50}$)/2, and particle size distribution (weight-based) is determined by the sieving method,
(c) the angle of repose of granules A is not greater than 38°, and said angle of repose is a value determined according to "Determination of the repose angle", the Japanese Pharmacopoeia, 17th edition, and
(d) the aspect ratio of granules A is at least 0.7, and said aspect ratio is an index representing the shape of a particle and determined by ISO 9276-6, and

wherein the content of the pharmaceutically active ingredient in the granules A represents at least 70 weight % of the weight of the plain granules of granules A.

5. The method for production according to 4 above, wherein the mean particle size of granules A is not greater than 250 μm.

6. The method for production according to 4 or 5 above, wherein the particle distribution index for granules A is 2.5 or less.

EFFECTS OF INVENTION

The present invention according to one of the above definitions enables easy production of an orally disintegrating tablet that possesses high hardness yet disintegrates very rapidly in the presence of water, employing various pharmaceutically active ingredient-containing granules, without regard to what the contained pharmaceutically active ingredient is. The present invention thus enables quick, steady, and easy provision of an orally disintegrating tablet that is advantageous both in its hardness and rapidness of disintegration, for a variety of drugs.

BRIEF DESCRIPTION OF DRAWINGS

[Fig. 1] Fig. 1 is a scanning electron micrograph (SEM) of the plain granules of granules A before coating in Example 1.
[Fig. 2] Fig. 2 is a scanning electron micrograph (SEM) of granules A in Example 1.
[Fig. 3] Fig. 3 is a scanning electron micrograph (SEM) of granules A in Example 3.
[Fig. 4] Fig. 4 is a scanning electron micrograph (SEM) of granules A in Comparative Example 1.
[Fig. 5] Fig. 5 is a scanning electron micrograph (SEM) of the plain granules of granules A before coating in Example 4.
[Fig. 6] Fig. 6 is a scanning electron micrograph (SEM) of granules A in Example 4.

DESCRIPTION OF EMBODIMENTS

In the present invention, the phrase "pharmaceutically active ingredient-containing granules" means granules comprehensively that contain a medical drug for a disease to be treated or prevented. Herein, a "pharmaceutically active ingredient" may be chosen as desired in accordance with a given disease that is to be treated or prevented, and is not limited to a particular, specific medical drug. This is because the essence of the present invention resides in a certain combination of structural and physical properties of granules.

The present invention is based on the discovery that a combination of both of high hardness and advantageous disintegrability in the presence of water is realized in an orally disintegrating tablet that is produced by tableting a mixture comprising pharmaceutically active ingredient-containing granules, which are independent of any specific compositions while possessing certain physical properties falling within a predetermined range, and at least a disintegrant. Therefore, there is no limitation as to specific components, including pharmaceutically active ingredient, that may build pharmaceutically active ingredient-containing granules.

For example, pharmaceutically active ingredient-containing granules may contain, in addition to a pharmaceutically active ingredient, any of additives that are pharmaceutically permitted, such as excipients, binders, and sweeteners. Specific examples of excipients mentioned above include D-mannitol, lactose, xylitol, maltose, maltitol, trehalose, sucrose, microcrystalline cellulose, corn starch, calcium hydrogen phosphate, anhydrous calcium hydrogen phosphate, and the like; and specific examples of above-mentioned binders include hypromellose, polyvinylpyrrolidone, polyvinylalcohol, carboxyvinyl polymers, carboxymethylethyl cellulose, hydroxyethyl cellulose, methacrylate copolymers, ethyl cellulose, aminoalkylmethacrylate copolymers, hydroxypropyl cellulose, methyl cellulose, pullulan, and the like, without limitation.

In the present invention, there is no particular limitation as to disintegrants employed, insofar as they do not reduce the effect of the present invention, and their specific examples include sugars such as sucrose, lactose, trehalose, and maltose; sugar alcohols such as D-mannitol, xylitol, and maltitol; starch or starch derivatives such as corn starch, sodium carboxymethyl starch (Primojel), and partly pregelatinized starch (PCS); celluloses such as microcrystalline cellulose, and low substituted hydroxypropyl cellulose; carmelloses such as carmellose, carmellose calcium, croscarmellose sodium (Ac-Di-Sol); crospovidone, calcium hydrogen phosphate, anhydrous calcium hydrogen phosphate, and the like, without limitation.

In the present invention, an orally disintegrating tablet may contain other pharmaceutically acceptable additives as needed, insofar as they do not reduce the effect of the present invention.

In the present invention, "a mixture comprising at least a disintegrant, and granules A containing a pharmaceutically active ingredient" may contain, in addition to granules A and a disintegrant, other conventional additives (excipients, binders, and sweeteners), and such additives may be in the form of powder, granules, or a mixture of them.

In the present invention, the mean particle size of the pharmaceutically active ingredient-containing granules (granules A) is not greater than 300 μm, more preferably not greater than 280 μm, and particularly preferably not greater than 250 μm.

In the above, "mean particle size" is defined as the 50% particle size ($D_{50}$) in particle size distribution (weight-based) determined by the sieving method. Besides, in Examples and Comparative Example described later, the particle size distribution of granules was determined on a sieve analyzer (continuous full-automatic sonic vibration sieve analyzer, Robot shifter RPS-205, mfd by Seisin Enterprise Co., Ltd.) using standard 8 sieves (22, 30, 42, 60, 83, 100, 140, 200-mesh) according to JIS Z8801.

In the present invention, the particle distribution index for granules A is 3.0 or less, more preferably 2.5 or less, still more preferably 2.0 or less, further more preferably 1.81 or less, and particularly preferably 1.7 or less.

Herein, "particle distribution index" is an index that represents the degree of extent of particle size distribution, and defied by the following formula. The closer to 1 a particle distribution index is, the closer to the mean particle size comes the particle distribution.

$$\text{Particle distribution index} = (D_{50}/D_{10} + D_{90}/D_{50})/2$$

In the above formula, "$D_{10}$" and "$D_{90}$" denote 10% particle size and 90% particle size, respectively, in the distribution as determined by the sieving method.

In the present invention, the angle of repose of granules A is not greater than 38 degrees, more preferably not greater than 36 degrees, and still more preferably not greater than 33 degrees.

Herein, "the angle of repose" is defined as the steepest angle relative to a horizontal plane at which the surface layer of piled granules on the horizontal plane can remain at rest. In the present invention, the angle of repose, more specifically, is a value determined according to "Determination of the repose angle", the Japanese Pharmacopoeia, 17th edition.

In the present invention, the content of the pharmaceutically active ingredient in granules A represents at least 70 weight % of the weight of the plain granules of granules A. Herein, the term "plain granule" means a portion of granule A excluding the coating layers in the case where granule A is a coated one, and if granule A is uncoated, the same meaning as granule A itself.

In the present invention, the proportion of granules A in the orally disintegrating tablet is preferably at least 25 weight %, and more preferably at least 30 weight %.

The present inventors found that the disintegration time of an orally disintegrating tablet tends to be shorter as the particle shape of granules A comes closer to a sphere. Therefore, it is preferable that the aspect ratio of the particles of granules A is somewhat large, and in the present invention the granules have aspect ratio of 0.7 or more, in production of orally disintegrating tablets.

In the present invention, "aspect ratio" is an index representing the shape of a particle and defined in ISO 9276-6 as "Feret's distance/ISO maximum length" of a particle. Herein, "Feret's distance" (or "minimum Feret's diameter") means the minimum distance between two parallel lines sandwiching the projected image of a photographed particle. And "ISO maximum length" means the length between 2 points chosen on the contour of the projected image of a particle so that their distance becomes maximum. Thus, the aspect ratio of a spherical particle is 1 (maximum). The aspect ratio of granules A is obtained by measuring Feret's distance and ISO maximum length of individual granules on the image of photographed sample granules spread on a measurement plate, calculating their respective aspect ratio, and statistically processing the collected data. For example, a scanning electron microscope proX PREMIUM II (JASCO International) may be employed for this purpose.

EXAMPLES

The present invention is described in further detail below with reference to Examples.

[Examples 1-2]

Orally disintegrating tablets of Examples 1 and 2 were produced using each material at a proportion so as to give tablets of the composition shown in the table below and by following the procedure described later.

[Table 1]

Table 1.                                (Composition/tablet)

| | | Example 1 | Example 2 |
|---|---|---|---|
| Granules A | Sertraline hydrochloride | 56 mg | 56 mg |
| | Additives for granulation | 14.0 mg | 14.0 mg |
| | Hypromellose | 4 mg | 4 mg |
| | Talc | 1 mg | 1 mg |
| | Aminoalkylmethacrylate copolymer E | 36 mg | 36 mg |
| | Talc | 9 mg | 9 mg |
| | Total | 120 mg | 120 mg |
| | | | |
| Mixed | Granule B | 230 mg | - |
| | Microcrystalline cellulose | - | 219.3 mg |
| | Crospovidone | - | 18 mg |
| | Sucralose | 2.8 mg | - |
| | Titanium dioxide | 3.6 mg | - |
| | Light anhydrous silicic acid | 0.9mg | - |
| | Flavor | trace | - |
| | Magnesium stearate | 2.7 mg | 2.7 mg |
| | Total | 240 mg | 240 mg |
| | | | |
| | Tablet weight | 360 mg | 360 mg |

(Production process)

1. Example 1

(1) Preparation of pharmaceutically active ingredient-containing granules (Granules A)

Sertraline hydrochloride and the additives for granulation consisting of D-mannitol and hydroxypropyl cellulose were formed into granules by mixing with addition of water, then dried and sized to give plain granules. The proportion of sertraline in the plain granules was 80 weight %. The plain granules then were put into a fluid bed granulator and sprayed with a suspension prepared by dispersing talc in a hypromellose aqueous solution to coat the plain granules, then dried and sized to give sized granules. The sized granules then were put into a fluid bed granulator and coated by spraying a suspension prepared by dispersing talc in a mixture liquid formed of aminoalkylmethacrylate copolymer E solution in anhydrous ethanol and purified water, and after washing the path with ethanol solution in water, the granules were dried, and sized to give pharmaceutically active ingredient-containing granules (granules A).

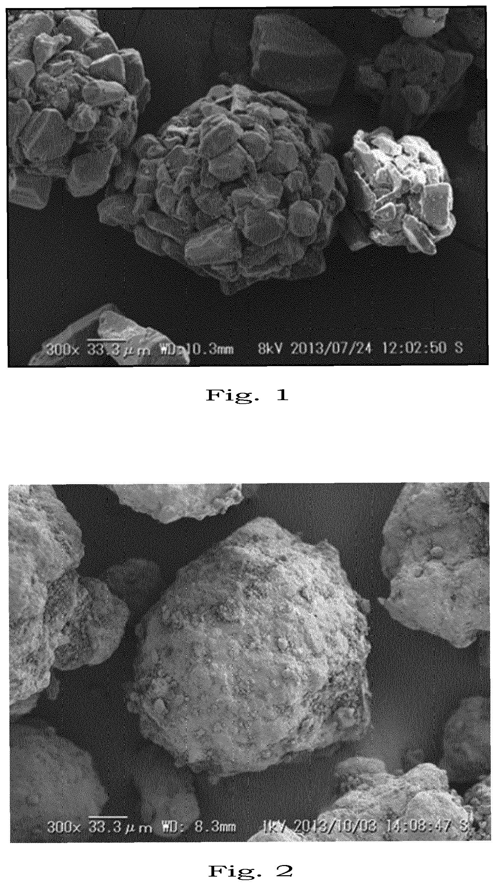

Granules A were measured for the angle of repose (°), and their particle size distribution was measured to derive the particle distribution index. The granules before coated into granules A (plain granules) were also measured for the particle size distribution to derive the particle distribution index. Further, the aspect ratio of granules A was measured using a scanning electron microscope proX PREMIUM II (JASCO International). Data of particle size and particle shape were collected and analyzed by automated image analysis. A scanning electron micrograph (SEM) of the plain granules, before coated into granules A, is shown in Fig. 1, and a scanning electron micrograph (SEM) of granules A in Fig. 2.

(2) Pharmaceutically active ingredient-free granules (granules B)

Using ingredients for granulation (containing disintegrant) consisting of D-mannitol, a cellulose compound, starch, crospovidone, and light anhydrous silicic acid, pharmaceutically active ingredient-free granules (granules B) were produced by fluid-bed granulation, drying, and sizing in a conventional manner.

(3) Mixing and tableting

Granules A, granules B, sucralose, titanium dioxide, light anhydrous silicic acid and a flavor were mixed, and to this mixture was further added magnesium stearate, mixed, and tableted under three different tableting pressures of 12 kN, 14 kN, and 16 kN, respectively, to give orally disintegrating tablets of Example 1.

2. Example 2

The same granules A as in Example 1 were employed. Granules A, microcrystalline cellulose, and crospovidone (disintegrant) were mixed, and to this mixture was further added magnesium stearate, mixed, and tableted under three different tableting pressures of 8 kN, 10 kN, and 12 kN, respectively, to give orally disintegrating tablets of Example 2.

(Examples 3, 3A-3D, and Comparative Example 1)

Orally disintegrating tablets of Example 3 and Comparative Example 1 were produced following the procedure described later using each material at a proportion so as to give tablets of the composition shown in following table.

[Table 2]

Table 2.            (Composition/tablet)

| | | Example 3 | Comparative Example 1 |
|---|---|---|---|
| Granules A | Irbesartan | 100 mg | 100 mg |
| | Additives 1 for granulation | 19.8 mg | 19.8 mg |
| | Additives 2 for granulation | 5 mg | 5 mg |
| | Sucralose | 10 mg | 10 mg |
| | Total | 134.8 mg | 134.8 mg |
| Mixed | Granules B | 186.5 mg | 186.5 mg |
| | Crospovidone | 16 mg | 16 mg |
| | Carmellose | 16 mg | 16 mg |
| | Light anhydrous silicic acid | 3.6 mg | 3.6 mg |
| | Flavor | trace | trace |
| | Magnesium stearate | 3.6 mg | 3.6 mg |
| | Total | 225.2 mg | 225.2 mg |
| Tablet weight | | 360 mg | 360 mg |

(Production process)

1. Example 3

(1) Pharmaceutically active ingredient-containing granules (granules A)

Additives 1 for granulation consisting of irbesartan, D-mannitol, croscarmellose sodium, and hypromellose were mixed with sucralose (sweetener), and formed into granules, which then were sprayed with an aqueous solution of additives 2 for granulation consisting of citric acid hydrate and hypromellose, dried, and sized to produce pharmaceutically active ingredient-containing granules (granules A). The proportion of irbesartan in granules A was 74.2 weight %.

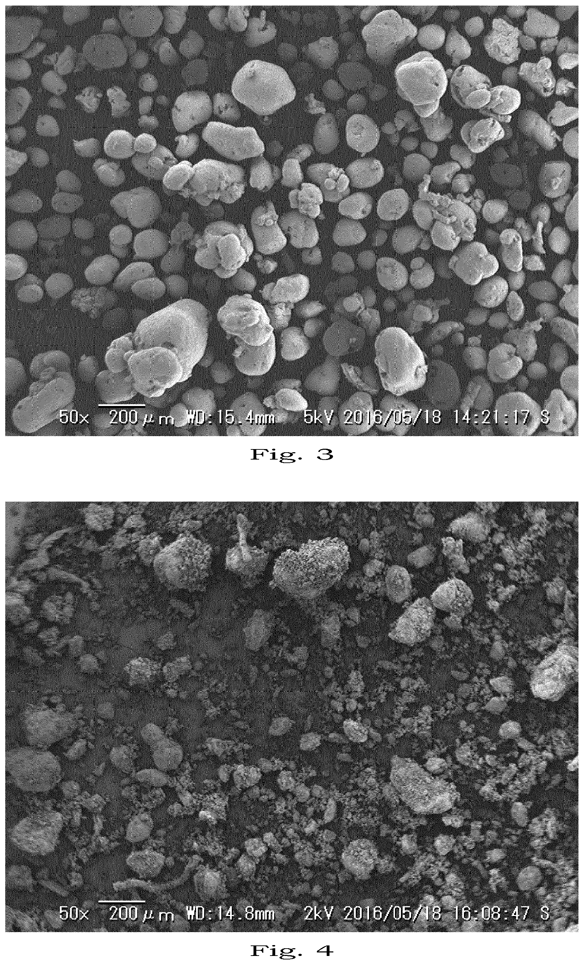

Granules A were measured for their repose angle (°), and their particle size to derive particle distribution index, as well as their aspect ratio. An electron micrograph (SEM) of granules A is shown in Fig. 3.

(2) Pharmaceutically active ingredient-free granules (granules B)

Employing ingredients for granules (containing disintegrant) consisting of D-mannitol, a cellulose compound, starch, crospovidone, and light anhydrous silicic acid, pharmaceutically active ingredient-free granules (granules B) were produced through fluid bed granulation, drying, and sizing according to a conventional manner.

(3) Mixing and tableting

Granules A, granules B, crospovidone, carmellose, light anhydrous silicic acid a flavor were mixed, and to this mixture was further added magnesium stearate, mixed, and tableted under the tableting pressure of 13 kN to produce orally disintegrating tablets of Example 3.

2. Examples 3A-3D

Granules A were produced in the same manner as in Example 3 except that the sizing conditions were replaced with four different ones, and they were measure for their angle of repose (°), particle size distribution to drive their particle distribution index, and for their aspect ratio as well. Using the granules A thus obtained, orally disintegrating tablets of Examples 3A to Examples 3D were produced, through addition of granules B, crospovidone, carmellose, light anhydrous silicic acid, and a flavor, further addition of magnesium stearate, mixing, and tableting as in Example 3, but setting the tableting pressure at two levels of 6 kN and 8 kN.

3. Comparative Example 1

Granules A were produced in the same manner as in granules A of Example 3 except that a modified sizing condition was employed so as to give an altered particle size distribution. Granules A were measured for their angle of repose (°), particle size distribution to drive their particle distribution index, and for their aspect ratio as well. An electron micrograph (SEM) of granules A is shown in Fig. 4.
Pharmaceutically active ingredient-free granules (granules B) were also produced in the same manner as in Example 3, and orally disintegrating tablets of Comparative Example 1 were produced using granules A, granules B, and the additives as in Example 3, and by tableting at a tableting pressure of 8 kN.

Example 4

Orally disintegrating tablets of Example 4 were produced using each material at a proportion so as to give tablets of the composition shown in the table below and by following the procedure described later.

[Table 3]

Table 3.

| | | | Example 4 |
|---|---|---|---|
| Granules A | Pregabalin | | 150 mg |
| | Additives for granulation | | 37.8 mg |
| | Aminoalkylmethacrylate copolymer E | | 75 mg |
| | Talc | | 37.5 mg |
| | Total | | 300.3 mg |
| | | | |
| Mixed | Granules B | | 475.2 mg |
| | D-mannitol | | 0.66 mg |
| | Aspartame | | 10.30 mg |
| | Magnesium stearate | | 5.54 mg |
| | Total | | 491.7 mg |
| | | | |
| | Tablet weight | | 792 mg |

(Production procedure)

(1) Production of pharmaceutically active ingredient-containing granules (granules A)

Pregabalin and additives for granulation consisting of corn starch and povidone were mixed and put into a fluid bed granulator, and sprayed with purified water, granulated, dried and sized to prepare plain granules. The proportion of pregabalin in the plain granules was 79.9 weight %. The plain granules were put into the fluid bed granulator, and coated by spraying them with a suspension prepared by dispersing talc in a solution of aminoalkylmethacrylate copolymer E (ethanol/water), dried, sized to prepare granules A. Granules A were measured for their angle of repose (°), and particle sized distribution to derive their particle distribution index, and for their aspect ratio as well. The granules before coated into granules A (plain granules) were also measured for their particle size distribution to drive their particle distribution index.

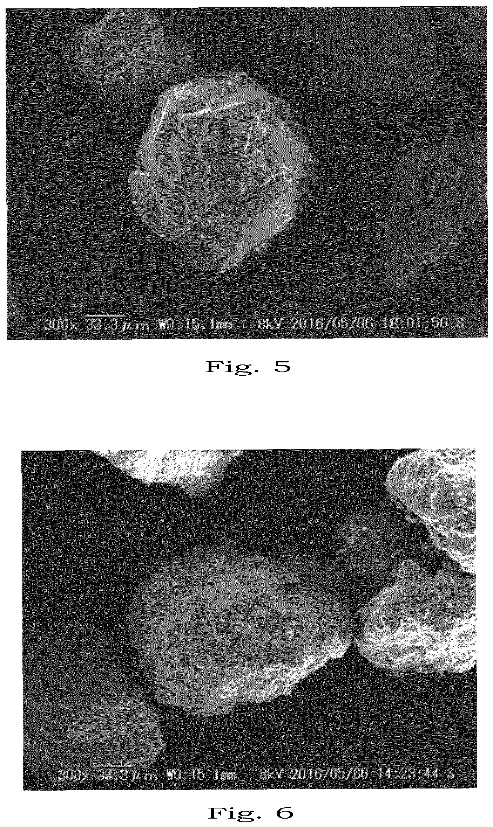

An electron micrograph (SEM) of the plain granules before coated into granules A is shown in Fig. 5, and an electron micrograph (SEM) of granules A is shown in Fig. 6.

(2) Pharmaceutically active ingredient-free granules (granules B)

Using ingredients for granules (containing disintegrant) consisting of D-mannitol, a cellulose compound, starch, crospovidone, and light anhydrous silicic acid, pharmaceutically active ingredient-free granules (granules B) were produced through granulation by fluid bed granulation, drying and sizing in a conventional manner.

(3) Mixing and tableting

Granules A, granules B, D-mannitol, and aspartame were mixed, and to this mixture was added magnesium stearate, mixed, and then tableted under three different tableting pressures of 12 kN, 14 kN, and 16 kN, respectively, to produce orally disintegrating tablets of Example 4.

(Evaluation)

Orally disintegration tablets of each of Examples and Comparative Example 1 were respectively measured for their hardness and disintegration time (Japanese Pharmacopoeia) corresponding each tableting pressure.

1. Disintegration time (Japanese Pharmacopoeia)

A disintegration testing apparatus (compliant with Japanese Pharmacopoeia) was employed. In a glass vessel was put 900 ml of water at 37 °C, and a basket (net-bottomed) containing tablets was moved up and down in the water contained in the vessel to measure the time required for the tablets to disintegrate completely.

2. Hardness

A hardness tester (TBH 425, ERWEKA) was employed. The apparatus was designed to convey a tablet to a jig, by which the tablet is squeezed on its lateral sides with a gradually increasing pressure to determine the load (N) at the time when the tablet was broken.

The results of measurement of the orally disintegrating tablets are shown in the following table, along with their angle of repose, particle size distribution, particle distribution index, and aspect ratio of corresponding granules A. As for Examples 1, 2 and 4, the particle size distribution and particle distribution index are also shown for corresponding plain granules.

[Table 4]

Table 4.

| | | | Example 1 | Example 2 | Example 3 | Example 3A | Example 3B | Example 3C | Example 3D | Comparative Example 1 | Example 4 |
|---|---|---|---|---|---|---|---|---|---|---|---|
| | Granules A | | Granules | Granules | Granules | Granules | Granules | Granules | Granules | Granules | Granules |
| | Granules B or powder | | Granules | Powder | Granules | Granules | Granules | Granules | Granules | Granules | Granules |
| Granules A | Angle of repose (°) | | 20 | 20 | 32 | 36 | 35 | 35 | 35 | 41 | 22 |
| | Particle size | D10 (μm) | 79.24 | 79.24 | 128.2 | 138.0 | 83.5 | 88.8 | 59.4 | 37.8 | 106 |
| | | D50 (μm) | 134.09 | 134.09 | 233.6 | 252.8 | 190.7 | 176 | 151.7 | 143.5 | 149 |
| | | D90 (μm) | 213.92 | 213.92 | 362.7 | 666.0 | 379.4 | 307.6 | 260.2 | 422.2 | 233 |
| | Particle distribution index | | 1.64 | 1.64 | 1.69 | 2.23 | 2.14 | 1.86 | 2.13 | 3.37 | 1.49 |
| | Aspect ratio | | 0.778 | 0.778 | 0.737 | 0.732 | 0.718 | 0.711 | 0.643 | 0.662 | 0.646 |
| Plain granules | Particle size | D10 (μm) | 49.66 | 49.66 | - | - | - | - | - | - | 71 |
| | | D50 (μm) | 85.89 | 85.89 | - | - | - | - | - | - | 97 |
| | | D90 (μm) | 162.31 | 162.31 | - | - | - | - | - | - | 165 |
| | Particle distribution index | | 1.81 | 1.81 | - | - | - | - | - | - | 1.54 |
| Tablets | Level 1 | Pressure (kN) | 12 | 8 | 13 | 6 | 6 | 6 | 6 | 8 | 12 |
| | | Hardness (N) | 88 | 82 | 82 | 58 | 77 | 84 | 91 | 98 | 89 |
| | | Disintegration time (sec) | 15 | 12 | 19 | 19 | 24 | 22 | 26 | 39 | 21 |
| | Level 2 | Pressure (kN) | 14 | 10 | - | 8 | 8 | 8 | 8 | - | 14 |
| | | Hardness (N) | 92 | 105 | - | 85 | 108 | 132 | 125 | - | 108.7 |
| | | Disintegration time (sec) | 17 | 13 | - | 23 | 28 | 29 | 29 | - | 23 |
| | Level 3 | Pressure (kN) | 16 | 12 | - | - | - | - | - | - | 16 |
| | | Hardness (N) | 120 | 117 | - | - | - | - | - | - | 116.7 |
| | | Disintegration time (sec) | 21 | 17 | - | - | - | - | - | - | 25 |

As seen from the results, the three versions of orally disintegrating tables of Example 1 possessed rapid disintegrability of 15 sec, 17 sec, and 21 sec despite that they had high hardness of 88 N, 92 N, and 120 N, respectively. The three versions of orally disintegrating tablets of Example 2 also possessed very rapid disintegrability of 12 sec, 13 sec, and 17 sec despite that they had high hardness of 82 N,105 N, and 117 N, respectively. Further, the orally disintegrating tablets of Example 3 and the three versions of orally disintegrating tablets of Example 4 also possessed advantageous disintegrability of 19 sec, 21 sec, 23 sec, and 25 sec despite that they had high hardness of 82 N, 89 N, 108.7 N, and 116.7 N.

Further, the orally disintegrating tablets of Comparative Example 1, despite of their relatively low hardness of 98 N, possessed a very long disintegration time of 39 sec, as compared with the hardness of 120 N with level 3 of Example 1, the hardness of 105 N and 117 N with levels 2 and 3, respectively, of Example 2, and the hardness of 108.7 N and 116.7 N of Example 4. This result indicates that the very compositions subjected to tableting in Examples confer notably advantageous disintegrability as compared with the composition of Comparative Example 1, even if they have comparable hardness.

Furthermore, in both Examples 1 and 2, the same granules A were commonly employed, and in the one (Example 1), other granules (granules B containing a disintegrant) and a lubricant were combined with them and tableted, whereas in the other (Example 2), additives (including a disintegrant), instead of granules B, were used just as a powder in the same amount, and tableted, both found to possess advantageous disintegrability. This indicates that the advantageous disintegrability with Examples has been brought about because of the property of granules A, irrespective of whether a disintegrant combined with granules A is in the from of granules (granules B) or in the form of a powder.

Still further, granules A in any of Examples possess high flowability exhibiting the angle of repose not more than 38°, and have their particle distribution index of not more than 3.0, showing their particle sizes rather concentrate near their mean particle size. In contrast, granules A in Comparative Example 1 exhibit a poor flowability showing a large angle of repose of 41°, and have their particle distribution index as large as 3.37, meaning a particle size distribution scattering in a broad range between small and large particles sizes. This indicates that the combination of a relatively small angle of repose and a small particle distribution index confers Examples' orally disintegrating tablets their characteristic properties, i.e, high hardness and advantageous disintegrability.

In addition, in Examples 3A-3D, which have the same composition, there is found a tendency among the tablets produced under the same tableting pressure, that the higher aspect ratio corresponds to the shorter disintegration time, and among them, Examples 3A-3C using granules A having their aspect ratio not less than 0.7 are found to be able to provide orally disintegrating tablets further advantageous as compared with Example 3D.

INDUSTRIAL APPLICABILITY

The present invention is useful as is enables steady and quick provision of orally disintegrating tablets having high hardness and excellent disintegrability in the presence of water with a wide variety of medical drugs orally administered.

**Claims**

1.　An orally disintegrating tablet as a compression molding product of a mixture comprising at least a disintegrant, and granules A containing a pharmaceutically active ingredient, wherein the proportion of granules A in the orally disintegrating tablet is at least 25 weight %, and
wherein

(a) the mean particle size of granules A is not greater than 300 $\mu$m, and said mean particle size of granules A is the 50% particle size ($D_{50}$) in particle size distribution (weight-based) determined by the sieving method,
(b) the particle distribution index for granules A is 3.0 or less, and particle distribution index = ($D_{50}/D_{10}$ + $D_{90}/D_{50}$)/2, and particle size distribution (weight-based) is determined by the sieving method,
(c) the angle of repose of granules A is not greater than 38°, and said angle of repose is a value determined according to "Determination of the repose angle", the Japanese Pharmacopoeia, 17th edition, and
(d) the aspect ratio of granules A is at least 0.7, and said aspect ratio is an index representing the shape of a particle and determined by ISO 9276-6 as "Feret's distance/ISO maximum length" of a particle, and

wherein the content of the pharmaceutically active ingredient in the granules A represents at least 70 weight % of the weight of the plain granules of granules A.

2.　The orally disintegrating tablet according to claim 1, wherein the mean particle size of granules A is not greater than

250 $\mu$m.

3. The orally disintegrating tablet according to claim 1 or 2, wherein the particle distribution index for granules A is 2.5 or less.

4. A method for production of an orally disintegrating tablet comprising compression molding a mixture comprising at least a disintegrant, and granules A containing a pharmaceutically active ingredient, wherein the proportion of granules A in the orally disintegrating tablet is at least 25 weight %, and wherein

(a) the mean particle size of granules A is not greater than 300 $\mu$m, and said mean particle size of granules A is the 50% particle size ($D_{50}$) in particle size distribution (weight-based) determined by the sieving method,
(b) the particle distribution index for granules A is 3.0 or less, and particle distribution index = ($D_{50}/D_{10}$ + $D_{90}/D_{50}$)/2, and particle size distribution (weight-based) is determined by the sieving method,
(c) the angle of repose of granules A is not greater than 38°, and said angle of repose is a value determined according to "Determination of the repose angle", the Japanese Pharmacopoeia, 17th edition, and
(d) the aspect ratio of granules A is at least 0.7, and said aspect ratio is an index representing the shape of a particle and determined by ISO 9276-6, and

wherein the content of the pharmaceutically active ingredient in the granules A represents at least 70 weight % of the weight of the plain granules of granules A.

5. The method for production according to claim 4, wherein the mean particle size of granules A is not greater than 250 $\mu$m.

6. The method for production according to claim 4 or 5, wherein the particle distribution index for granules A is 2.5 or less.

**Patentansprüche**

1. Oral zerfallende Tablette als Formpressprodukt aus einem Gemisch, das mindestens ein Sprengmittel und Körnchen A, die einen pharmazeutisch wirksamen Bestandteil enthalten, umfasst, wobei der Anteil der Körnchen A in der oral zerfallenden Tablette mindestens 25 Gew.-% beträgt, und wobei

(a) die mittlere Partikelgröße der Körnchen A nicht größer als 300 $\mu$m ist und die mittlere Partikelgröße der Körnchen A die 50%-Partikelgröße ($D_{50}$) in der mit dem Siebverfahren bestimmten Partikelgrößenverteilung (gewichtsbasiert) ist,
(b) der Partikelverteilungsindex für die Körnchen A 3,0 oder kleiner ist und Partikelverteilungsindex = ($D_{50}/D_{10}$ + $D_{90}/D_{50}$)/2 und die Partikelgrößenverteilung (gewichtsbasiert) mit dem Siebverfahren bestimmt wird,
(c) der Schüttwinkel der Körnchen A nicht größer als 38° ist und der Schüttwinkel ein gemäß "Bestimmung des Schüttwinkels", Japanisches Arzneibuch, 17. Ausgabe, bestimmter Wert ist und
(d) das Aspektverhältnis der Körnchen A mindestens 0,7 beträgt und das Aspektverhältnis ein Index ist, der die Form eines Partikels repräsentiert und nach ISO 9276-6 als "Feret-Abstand/maximale Länge nach ISO" eines Partikels bestimmt wird und

wobei der Gehalt des pharmazeutisch wirksamen Bestandteils in den Körnchen A mindestens 70 Gew.-% des Gewichts der puren Körnchen der Körnchen A ausmacht.

2. Oral zerfallende Tablette nach Anspruch 1, wobei die mittlere Partikelgröße der Körnchen A nicht größer als 250 $\mu$m ist.

3. Oral zerfallende Tablette nach Anspruch 1 oder 2, wobei der Partikelverteilungsindex für die Körnchen A 2,5 oder kleiner ist.

4. Verfahren zur Herstellung einer oral zerfallenden Tablette, umfassend Formpressen eines Gemisches, das mindestens ein Sprengmittel und Körnchen A, die einen pharmazeutisch wirksamen Bestandteil enthalten, umfasst, wobei der Anteil der Körnchen A in der oral zerfallenden Tablette mindestens 25 Gew.-% beträgt, und wobei

(a) die mittlere Partikelgröße der Körnchen A nicht größer als 300 $\mu$m ist und die mittlere Partikelgröße der Körnchen A die 50%-Partikelgröße ($D_{50}$) in der mit dem Siebverfahren bestimmten Partikelgrößenverteilung (gewichtsbasiert) ist,

(b) der Partikelverteilungsindex für die Körnchen A 3,0 oder kleiner ist und Partikelverteilungsindex = ($D_{50}/D_{10}$ + $D_{90}/D_{50}$)/2, und die Partikelgrößenverteilung (gewichtsbasiert) mit dem Siebverfahren bestimmt wird

(c) der Schüttwinkel der Körnchen A nicht größer als 38° ist und der Schüttwinkel ein gemäß "Bestimmung des Schüttwinkels", Japanisches Arzneibuch, 17. Ausgabe, bestimmter Wert ist und

(d) das Aspektverhältnis der Körnchen A mindestens 0,7 beträgt und das Aspektverhältnis ein Index ist, der die Form eines Partikels repräsentiert und nach ISO 9276-6 bestimmt wird und

wobei der Gehalt des pharmazeutisch wirksamen Bestandteils in den Körnchen A mindestens 70 Gew.-% des Gewichts der puren Körnchen der Körnchen A ausmacht.

**5.** Verfahren zur Herstellung nach Anspruch 4, wobei die mittlere Partikelgröße der Körnchen A nicht größer als 250 $\mu$m ist.

**6.** Verfahren zur Herstellung nach Anspruch 4 oder 5, wobei der Partikelverteilungsindex für die Körnchen A 2,5 oder kleiner ist.

## Revendications

**1.** Comprimé orodispersible comme produit de moulage par compression d'un mélange comprenant au moins un désintégrant, et des granulés A contenant un ingrédient pharmaceutiquement actif, la proportion de granulés A dans le comprimé orodispersible étant d'au moins 25 % en poids, et

(a) la taille de particule moyenne des granulés A est de pas plus de 300 $\mu$m, et ladite taille de particules moyenne des granulés A est la taille de particule de 50 % (D50) dans la distribution de taille de particule (basée sur le poids) déterminée par le procédé de tamisage,

(b) l'indice de distribution de particule pour les granulés A est de 3,0 ou moins, et l'indice de distribution de particule = ($D_{50}/D_{10}$ + $D_{90}/D_{50}$) /2 et la distribution de taille de particule (basée sur le poids) est déterminée par le procédé de tamisage,

(c) l'angle de repos des granulés A est de pas plus de 38°, et ledit angle de repos est une valeur déterminée selon « Détermination de l'angle de repos », la pharmacopée japonaise 17ème édition, et

(d) le rapport d'aspect des granulés A est d'au moins 0,7, et ledit rapport d'aspect est un indice représentant la forme d'une particule et déterminé par la norme ISO 9276-6 comme « distance de Feret/longueur maximum ISO » d'une particule, et

dans lequel la teneur de l'ingrédient pharmaceutiquement actif dans les granulés A représente au moins 70 % en poids du poids des granulés pleins de granulés A.

**2.** Comprimé orodispersible selon la revendication 1, dans lequel la taille de particule moyenne des granulés A est de pas plus de 250 $\mu$m.

**3.** Comprimé orodispersible selon la revendication 1 ou 2, dans lequel l'indice de distribution de particule pour les granulés A est de 2,5 ou moins.

**4.** Procédé de production d'un comprimé orodispersible comprenant le moulage par compression d'un mélange comprenant au moins un désintégrant, et des granulés A contenant un ingrédient pharmaceutiquement actif, la proportion de granulés A dans le comprimé orodispersible étant d'au moins 25 % en poids, et

(a) la taille de particule moyenne des granulés A étant de pas plus de 300 $\mu$m, et ladite taille de particules moyenne des granulés A est la taille de particule de 50 % (D50) dans la distribution de taille de particule (basée sur le poids) déterminée par le procédé de tamisage,

(b) l'indice de distribution de particule pour les granulés A est de 3,0 ou moins, et l'indice de distribution de particule = ($D_{50}/D_{10}$ + $D_{90}/D_{50}$) /2 et la distribution de taille de particule (basée sur le poids) est déterminée par le procédé de tamisage,

(c) l'angle de repos des granulés A étant de pas plus de 38°, et ledit angle de repos est une valeur déterminée selon « Détermination de l'angle de repos », la pharmacopée japonaise 17ème édition, et
(d) le rapport d'aspect des granulés A étant d'au moins 0,7, et ledit rapport d'aspect est un indice représentant la forme d'une particule et déterminé par la norme ISO 9276-6, et

dans lequel la teneur de l'ingrédient pharmaceutiquement actif dans les granulés A représente au moins 70 % en poids du poids des granulés pleins des granulés A.

5. Procédé de production selon la revendication 4, dans lequel la taille de particule moyenne des granulés A est de pas plus de 250 μm.

6. Procédé de production selon la revendication 4 ou 5, dans lequel l'indice de distribution de particule pour les granulés A est de 2,5 ou moins.

Fig. 1

Fig. 2

50x ⎯⎯ 200 μm WD:15.4mm 5kV 2016/05/18 14:21:17 S

Fig. 3

50x ⎯⎯ 200 μm WD:14.8mm 2kV 2016/05/18 16:08:47 S

Fig. 4

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S5824410 B
- JP H06502194 B
- WO 9520380 A
- JP 4551627 B
- JP 5584509 B
- JP 5062761 B
- JP 5062872 B
- JP 4446177 B
- EP 2810659 A
- EP 1203580 A

**Non-patent literature cited in the description**

- Pharmaceutical dosage forms: tablets. Informa Healthcare, 2008, vol. 1, 77-78
- Determination of the repose angle. Japanese Pharmacopoeia